(19) **Europäisches Patentamt**
**European Patent Office**
**Office européen des brevets**

(11) **EP 4 678 282 A1**

(12) **EUROPEAN PATENT APPLICATION**
published in accordance with Art. 153(4) EPC

(43) Date of publication:
**14.01.2026 Bulletin 2026/03**

(21) Application number: **24767097.9**

(22) Date of filing: **04.03.2024**

(51) International Patent Classification (IPC):
**B01J 20/282** (2006.01)    **B01J 20/285** (2006.01)
**G01N 30/88** (2006.01)

(52) Cooperative Patent Classification (CPC):
**B01J 20/282; B01J 20/285; G01N 30/88**

(86) International application number:
**PCT/JP2024/007973**

(87) International publication number:
**WO 2024/185729 (12.09.2024 Gazette 2024/37)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **08.03.2023 JP 2023036046**

(71) Applicant: **JNC Corporation
Chiyoda-ku
Tokyo 100-8105 (JP)**

(72) Inventors:
• **TAKIZAWA, Kazuhiro
Yokohama-shi, Kanagawa 236-8605 (JP)**

• **MATSUMOTO, Yoshihiro
Yokohama-shi, Kanagawa 236-8605 (JP)**
• **IWAMOTO, Eri
Yokohama-shi, Kanagawa 236-8605 (JP)**
• **KADOI, Kenji
Yokohama-shi, Kanagawa 236-8605 (JP)**
• **NAKAMA, Tsuyoshi
Yokohama-shi, Kanagawa 236-8605 (JP)**

(74) Representative: **Becker, Eberhard
Becker Kurig & Partner
Patentanwälte mbB
Bavariastraße 7
80336 München (DE)**

(54) **POROUS PARTICLES, LIQUID CHROMATOGRAPHY CARRIER, LIQUID CHROMATOGRAPHY APPARATUS, AND METHOD FOR SEPARATING/PURIFYING BIOPOLYMER**

(57)    The purpose of the present invention is to provide a technology for performing purification of a biomedicine or the like at a high processing speed using a conventional apparatus in which a column is filled with a filler.

Provided are porous particles which are characterized in that each of the porous particles has an approximately spherical shape and has a communicating pore structure composed of a three-dimensional network-like framework and a void formed in the framework, wherein, in each of the porous particles, the communicating pore penetrates through from the surface of the particle to the inside of the particle. The porous particles are also characterized in that, when a column having an inner diameter of 0.78 cm and a length of 30 cm is filled with the porous particles and a slurry containing 5 mg/mL of silica fine particles each having a particle diameter of 100 nm is passed through the column at a column temperature of 25°C and a flow rate of 0.4 mL/ min using pure water as a mobile phase, the retention time of the silica fine particles is 15.0 minutes or more.

FIG. 1

**Description**

Technical Field

The invention relates to porous particles with a large pore diameter, and a chromatography technology using the porous particles at a high processing speed.

Related Art

In the chromatography used for separation and purification of biomedicines, etc., porous particles can be utilized as a material with high binding capacity due to having a large surface area. In recent years, efforts have been made to arrange the pores in porous particles as continuous pores so that the voids of interconnected pores inside the particles can also be used as adsorption sites (Patent Documents 1, 2, 3). Additionally, while conventional porous particles are optimized for separating biomolecules such as antibodies, proteins, and nucleic acids with sizes typically in the range of 5 nm to 10 nm, efforts have been made to increase the pore diameter of porous particles, thereby allowing large particles to be adsorbed in the pores, so as to increase the binding capacity for larger particles, such as viruses (Patent Document 4).

In recent years, to improve the productivity of biomedicines, etc., the ability to bind a target substance per unit time has received attention. Such ability is expressed as the amount of the target substance bound per unit volume per unit time (mg/mL/min), which leads to a decrease in the chromatography adsorption material used for separation and purification and/or a decrease in the time required for separation and purification. Since the diffusion of the particles of the target substance into pores to bind to conventional porous particles is rate-controlled, there is an issue that, especially at a high processing speed with which the residence time is less than 1 minute, when the flow rate is increased to decrease the residence time into the column into which particles are filled, the binding capacity of the target substance to the particles decreases significantly. Therefore, increasing the flow rate does not lead to increased productivity. As a method to solve the issue of having a decreased binding capacity at a high processing speed, chromatography purification methods using membranes, monoliths, or fibers instead of porous particles are known. With a liquid passing between widely opened fibers in such material, the diffusion rate control becomes less susceptible, which is a property that can reduce the flow rate dependence of the binding capacity. However, membranes, monoliths, and fibers cannot be filled into columns that have been conventionally used, and an issue that a specialized container is required arises. Additionally, when using membranes, monoliths, or fibers, there is an issue that the pressure increases rapidly at the time of clogging.

Citation List

Patent Document(s)

[Patent Document 1] International Publication No. 2016/013568
[Patent Document 2] International Publication No. 2017/026424
[Patent Document 3] Japanese Patent Application Publication No. 2020-026511
[Patent Document 4] Japanese Patent Application Publication No. 2022-515769
[Patent Document 5] International Publication No. 2015/029790

[Non-patent Document(s)]

[Non-patent Document 1] W.M. Deen, F.G. Smith III, J. Membr. Sci. 1982, 12, 217-237
[Non-patent Document 2] P DePhillips , A M Lenhoff, J Chromatogr A. 2000, 883, 39-54

SUMMARY OF INVENTION

Technical Problem

As described above, in order to facilitate the separation and purification efficiency of biomedicines, etc., there is a demand for a purification method that can improve the amount of target substance bound per unit volume, per unit time (productivity) while utilizing conventional equipment such as filled columns, and that has a small risk of clogging.

Therefore, an issue of the invention is to provide a technique for performing purification of a biomedicine, etc., at a high processing speed using a conventional apparatus in which a column is filled with a filler.

Solution to Problem

As a result of intensive research to solve the issues, the inventors have discovered porous particles having a specific interconnected pore structure formed by a three-dimensional network skeleton and voids thereof, and having larger interconnected pores. By using the porous particles as a liquid chromatography carrier, it is found that a high adsorption capacity can be maintained even under a high flow rate condition. Accordingly, the invention is completed.

That is, the invention includes the following.

[1] A porous particle is provided.

The porous particle is substantially spherical and comprises an interconnected pore structure formed by a three-dimensional network skeleton and voids thereof, and the interconnected pores penetrate from a particle surface to an interior.
When the porous particle is filled in a column with an inner diameter of 0.78 cm and a length of 30 cm and a slurry containing 5 mg/mL of silica fine particles with a particle diameter of 100 nm is passed through using pure water as a mobile phase at a column temperature of 25°C and a flow rate of 0.4 mL/min, a retention time of the silica fine particles is 15.0 minutes or more.

[2] In the porous particle according to [1], a porous diameter is 650 nm or more.
[3] A porous particle modified by a ligand is provided.

The porous particle is substantially spherical and comprises an interconnected pore structure formed by a three-dimensional network skeleton and voids thereof, and the interconnected pores penetrate from a particle surface to an interior.
A porous diameter is 400 nm or more.

[4] In the porous particle according to any one of [1] to [3], a main component is cellulose acetate or cellulose.
[5] In the porous particle according to any one of [1] to [4], a median diameter is 40 $\mu$m to 200 $\mu$m.
[6] In the porous particle according to any one of [1] to [5], a specific surface area according to a BET multipoint method is 1 $m^2$/g to 200 $m^2$/g.
[7] In the porous particle according to [1] or [2], the porous particle according to any one of [1], [2] and [4] to [6] is modified by a ligand.
[8] In the porous particle according to [3] or [7], the ligand is one or more selected from a group consisting of a ligand containing an ion exchange group, an affinity ligand, and a ligand containing a hydrophobic group.
[9] A liquid chromatography carrier includes the porous particle according to any one of [1] to [8].
[10] A liquid chromatography apparatus includes a separation column into which the liquid chromatography carrier according to [9] is filled.
[11] A separation and purification method of a biopolymer includes:
a step of separating and purifying the biopolymer by using the liquid chromatography apparatus according to [10].
[12] In the separation and purification method according to [11], a size of the biopolymer is 1 nm of 100 nm.
[13] In the separation and purification method according to [11] or [12], the biopolymer is an antibody.

Effects of Invention

According to the invention, when separating and purifying a target substance such as a biopolymer, a large adsorption capacity can be achieved even under a high flow rate condition.

BRIEF DESCRIPTION OF DRAWINGS

[FIG. 1] FIG. 1 is a photograph (1000x magnification) of the appearance of particles manufactured in Manufacturing Example 1.
[FIG. 2] FIG. 2 is a photograph (1500x magnification) of the appearance of particles manufactured in Manufacturing Example 2.
[FIG. 3] FIG. 3 is a photograph (1300x magnification) of the appearance of particles manufactured in Manufacturing Example 3.

[FIG. 4] FIG. 4 is a photograph (800x magnification) of an internal cross-section of particles manufactured in Manufacturing Example 1.

[FIG. 5] FIG. 5 is a photograph (700x magnification) of an internal cross-section of particles manufactured in Manufacturing Example 2.

[FIG. 6] FIG. 6 is a graph showing properties when particles manufactured in Examples 1 to 4 and Comparative Examples 1 to 6 are filled in columns, and shows a 10% dynamic adsorption capacity of $\gamma$-globulin per unit time and per unit volume at a column residence time of 7.5 seconds in the case where the particles manufactured in Examples 1 to 4 and Comparative Examples 1 to 6 are used.

DESCRIPTION OF EMBODIMENTS

The shape of the porous particles of the invention is substantially spherical. The term "substantially spherical" as used herein means, for example, that the major diameter (the longest diameter) is not more than twice the minor diameter (the shortest diameter). The porous particles of the invention preferably have a major diameter and a minor diameter that are close to the same length, and more preferably are true spherical in shape.

The porous particle of the invention has an interconnected pore structure formed by a three-dimensional network skeleton and voids thereof, and the interconnected pores penetrate from the particle surface to the interior. Whether the particle has a three-dimensional network skeleton and interconnected pores can be determined, for example, from the measurement results of confocal laser microscope observation or scanning electron microscope (SEM) observation on the cross-section of the particle. Also, the fact that the interconnected pores penetrate from the particle surface to the interior can be confirmed, for example, by using the photograph of the internal cross-section of the porous particle of the invention. In the porous particle of the invention, the interconnected pores are not blocked on the particle surface but are open on the particle surface. By having such interconnected pore structure, the voids of the interconnected pores in the interior of the particles, in addition to the particle surface of the porous particles, can be used as adsorption sites for the target substance. Accordingly, the separation and purification efficiency of the target substance is facilitated.

The phrase "the interconnected pores penetrate from the particle surface to the interior" includes not only the state where the interconnected pore passes from an opening on the particle surface to a surface other than the particle surface through the particle interior (i.e., a state where both ends of the interconnected pore extend to the particle surface), but also the state where the interconnected pore extends from the particle surface to the interior but does not extend from the interior to a surface other than that particle surface (i.e., a state where only one end of the interconnected pore extends to the particle surface). Also, the interconnected pore extending from an opening on the particle surface to the interior may form a void in the particle interior continuously with one or more other interconnected pores.

The porous particles of the invention satisfy one or more of properties (1) to (2) as follows.

(1) When the porous particles are filled in a column with an inner diameter of 0.78 cm and a length of 30 cm, and a slurry containing 5 mg/mL of silica fine particles with a particle diameter of 100 nm is passed through by using pure water as a mobile phase at a column temperature of 25°C and a flow rate of 0.4 mL/min, the retention time of the silica fine particles is 15.0 minutes or more.

(2) The pore diameter of the porous particle in a state modified with a ligand is 400 nm or more.

(1) When the porous particles are filled in a column with an inner diameter of 0.78 cm and a length of 30 cm, and a slurry containing 5 mg/mL of silica fine particles with a particle diameter of 100 nm is passed through by using pure water as a mobile phase at a column temperature of 25°C and a flow rate of 0.4 mL/min, the retention time of the silica fine particles is 15.0 minutes or more, preferably 15.5 minutes or more, and more preferably 15.8 minutes or more. Also, the upper limit is preferably 30.0 minutes or less, more preferably 25.0 minutes or less, and even more preferably 20.0 minutes or less. Here, the porous particles filled in the column are porous particles in a state without ligand modification.

Specifically, as a method for filling the porous particles in a column, the porous particles are dispersed in pure water to form a slurry, then filled in a column (for example, a stainless steel column (manufactured by Tosoh Corporation)), and further compressed by flowing pure water at a flow rate of 0.4 mL/min for 1 hour or more. After that, a slurry containing 5 mg/mL of silica fine particles with a particle diameter of 100 nm is passed through the column at a flow rate of 0.4 mL/min. Pure water is used as the dispersion medium for the slurry containing 5 mg/mL of silica fine particles and as the mobile phase during retention time measurement. Also, the column temperature is adjusted to 25°C. As the silica fine particles with a particle diameter of 100 nm, commercially available silica fine particles, such as sicaster (manufacturing number: 43-00-102, manufactured by micromod), can be used.

"retention time" refers to the time from when a sample is injected into a column until the elution peak appears. The elution peak can be determined, for example, from the maximum value of RI detection intensity by using an HPLC apparatus (1260 Infinity, manufactured by Agilent Technologies).

In the case where the retention time of the silica fine particles is 15.0 minutes or more, the dynamic adsorption capacity of the target substance such as a biopolymer can be maintained at a high value even under a high flow rate condition.

In the field of chromatography, when evaluating the pore diameter of the porous particle, it is common to perform measurements using dextran with a molecular weight of 2 million as the exclusion limit molecular weight (Patent Document 5). This is under the premise that dextran with a molecular weight of 2 million cannot enter the interior of the porous particle. While dextran and polyethylene oxide are soft materials and can deform while moving through liquid and entering the pores of the particle, inorganic fine particles such as silica are rigid spheres that cannot deform and thus possess properties that make them difficult to enter the pores of the particle (Non-Patent Document 1). Therefore, in general, compared to dextran with a molecular weight of 2 million or polyethylene oxide having a molecular weight of 780,000 and a Stokes diameter exceeding 40 nm, silica fine particles with a smaller particle diameter of 30 nm have a shorter retention time. Here, when the porous particles of the invention are filled in a column, the retention time of silica fine particles with a particle diameter of 100 nm, which are rigid spheres and particles with a larger particle diameter, is 15.0 minutes or more, longer than conventional porous particles. This indicates that the porous particle of the invention has interconnected pores with a pore diameter large enough for even silica fine particles with a particle diameter of 100 nm, which are rigid spheres, to enter the interior of the particle.

Also, it is generally known that when spherical porous particles are filled in a column, the interparticle porosity becomes 30% to 40% (Non-Patent Document 2). The interparticle porosity herein is the total volume of voids between particles in the column interior divided by the total volume of the column. For example, assuming that a sample passes only through the voids between particles filled in a column with an inner diameter of 0.78 cm and a length of 30 cm, the retention time at a flow rate of 0.4 mL/min is theoretically 10.7 minutes to 14.3 minutes, and such time is the retention time for excluded particles such as silica fine particles. The porous particles of the invention have a retention time of 15.0 minutes or more for silica fine particles with a particle diameter of 100 nm. Thus, from the comparison with the excluded particle retention time, it is also shown that the porous particle of the invention has interconnected pores with a pore diameter large enough for silica fine particles with a particle diameter of 100 nm to enter the interior of the particle, in addition to the voids between particles.

The pore diameter of the porous particles of the invention is preferably 650 nm or more. Here, the porous particles refer to those in a state without ligand modification.

In the case of evaluating the pore diameter of particles with a large pore diameter like the porous particle of the invention, it is difficult to prepare excluded particles that do not enter the pores. Therefore, it is also effective to assume the excluded particle retention time from the theoretical retention time and use the excluded particle retention time for estimating the pore diameter when comparing the size of pore diameters between particles.

That is, when estimated by assuming the excluded particle retention time from the theoretical retention time, the pore diameter of the porous particle in a state without ligand modification is preferably 650 nm or more, more preferably 800 nm or more, and even more preferably 1000 nm or more. The upper limit is preferably 2000 nm or less, more preferably 1800 nm or less, and even more preferably 1700 nm or less.

The estimated value of the pore diameter can be calculated, for example, based on the retention time of polyethylene oxide according to the following procedure.

The porous particle of the invention in a state not modified by a ligand is filled in a column with an inner diameter of 0.78 cm and a length of 30 cm, and the retention time of polyethylene oxide is measured when a solution containing 5 mg/mL of polyethylene oxide is passed through using pure water as the mobile phase at a column temperature of 25°C and a flow rate of 0.4 mL/min.

Specifically, as a method for filling the porous particles in a column, the porous particles are dispersed in the pure water to form a slurry, then filled in a column (for example, a stainless steel column (manufactured by Tosoh Corporation)), and further compressed by flowing pure water at a flow rate of 0.4 mL/min for 1 hour or more. After that, each solution or slurry containing 5 mg/mL of polyethylene glycol with a weight average molecular weight of 106, a measurement sample (standard polyethylene oxide), and excluded particles (for example, silica fine particles with a particle diameter of 100 nm) are passed through the column at a flow rate of 0.4 mL/min. Pure water is used as the solvent or dispersion medium for each solution or slurry, and as the mobile phase during retention time measurement. Also, the column temperature is adjusted to 25°C. The value at which the RI detection intensity reaches the maximum is taken as the elution peak, and the time is considered as the retention time. As a measuring device, for example, a 1260 Infinity HPLC apparatus (manufactured by Agilent Technologies) can be used.

(i) The total volume $V_T$ (mL) of the mobile phase is calculated by the formula "retention time of polyethylene glycol with a weight average molecular weight of 106 (min) × flow rate (mL/min) × column volume (mL)". As polyethylene glycol with a weight average molecular weight of 106, for example, PEG10[6] manufactured by Agilent Technologies can be used.

(ii) The void volume $V_0$ (mL) between particles is calculated by the formula "excluded particle retention time (min) $\times$ flow rate (mL/min) $\times$ column volume (mL)".

The excluded particle retention time is obtained. As excluded particles, for example, commercially available silica fine particles with a particle diameter of 100 nm, such as sicaster (manufacturing number: 43-00-102, manufactured by micromod), can be used.

In the case of a porous particle with a small pore diameter, polymers or silica fine particles with a large molecular weight can be treated as excluded particles that do not enter the pores, so the excluded particle retention time can be determined from the retention time of the polymers or silica fine particles with a large molecular weight. However, in the case of a porous particle with a large pore diameter, even polymers or silica fine particles with a large molecular weight can enter the pores, making it difficult to calculate the excluded particle retention time.

Here, it is generally known that in the case where spherical porous particles are filled in a column, the interparticle porosity becomes 30% to 40% (Non-Patent Literature 1). Assuming that the sample passes only through the gaps between the particles filled in a column with an inner diameter of 0.78 cm and a length of 30 cm, the retention time at a flow rate of 0.4 mL/min is theoretically 10.7 minutes to 14.3 minutes. As described later, the particles in Manufacturing Examples 4 to 6 have relatively small pore diameters, so it is presumed that rigid spherical silica fine particles with a particle diameter of 100 nm hardly enter the pores. The retention times of the 100 nm silica fine particles in Manufacturing Examples 4-6 are all within the range of 13 minutes to 14 minutes. Therefore, based on the average of the retention times of silica fine particles in Manufacturing Examples 4 to 6, the value of the excluded particle retention time is fixed at 13.6 minutes to calculate $V_0$.

(iii) The elution volume $V_R$ (mL) of the measurement sample is calculated by the formula "retention time (min) of the measurement sample $\times$ flow rate (mL/min) $\times$ column volume (mL)". As measurement samples, for example, polyethylene oxides with weight average molecular weights of $2.36 \times 10^4$ Da (SE-2), $3.76 \times 10^4$ Da (SE-5), $1.07 \times 10^5$ Da (SE-8), $1.49 \times 10^5$ Da (SE-15), $2.8 \times 10^5$ Da (SE-30), $5.8 \times 10^5$ Da (SE-70), and $7.86 \times 10^5$ Da (SE-150) manufactured by Tosoh can be used. Also, polyethylene glycols with weight average molecular weights of 20440 Da (PEG21300), 15260 Da (PEG16100), 7460 Da (PEG7830), 4110 Da (PEG4040), 1020 Da (PEG1010), 620 Da (PEG610), and 400 Da (PEG400) manufactured by Agilent Technologies can be used.

(iv) The distribution coefficient $K_d$ is calculated by the following formula.

$$K_d = (V_R - V_0)/(V_T - V_0)$$

(v) Referring to "The Hydrodynamic Radii of Macromolecules and Their Effect on Red Blood Cell Aggregation" (J. K. Armstrong, et al., Biophysical Journal, 2004, 87, 4259-4270), the viscosity radius $r_s$ (nm) of each measurement sample is calculated based on the weight average molecular weight $M_w$ (Da) and the intrinsic viscosity $[\eta]$ (mL/g) by using the following formula.

$$r_s = (3[\eta]M_w/10_\pi N)^{1/3} \times 10^7$$

[In the formula, N represents Avogadro's number.]

(vi) Assuming a single pore diameter, the distribution coefficient $K_d$ and the hydraulic radius $r_{hyd}$ of the particle are expressed by the following formula.

$$K_d = (1 - r_s/r_{hyd})^2$$

By taking 0.5 power of $K^d$ when each measurement sample is measured as the vertical axis and the viscosity radius $r_s$ of each measurement sample as the horizontal axis and obtaining $- 1/r_{hyd}$ from the slope during linear approximation based on $K_d^{0.5} = 1 - r_s/r_{hyd}$, there is a distribution of pore diameters in actual porous particles, and a significant difference in slope exists between the case where the viscosity radius of the measurement sample is 5 nm or less (e.g., when the measurement sample is PEG21300 to PEG400) and the case where the viscosity radius is greater than 5 nm (e.g., when the measurement sample is SE-150 to SE-2). Therefore, in order to calculate the pore diameter of large pores that affect the adsorption of biopolymers, the 0.5 power of $K_d$ obtained by measuring each sample of the standard polyethylene oxide with a viscosity radius of 5 nm or more is taken as the vertical axis, and the viscosity radius $r_s$ of each sample is taken as the horizontal axis, and $-1/r_{hyd}$ is obtained from the slope during linear approximation based on the following formula.

$$K_d^{0.5} = C - r_s/r_{hyd}$$

[In the formula, C represents the intercept during linear approximation.]

(vii) Assuming that the pores of the particle are cylindrical through-holes, the pore diameter $r_{pore}$ and the hydraulic radius $r_{hyd}$ of the particle are represented by the following formula.

$$r_{pore} = r_{hyd} \times 2$$

From the formula, the pore diameter $r_{pore}$ of the particle is calculated as an estimated value of the pore diameter of the particle.

The particle internal porosity $\varepsilon_p$ (porosity inside the particle) of the porous particle in a state not modified by the ligand of the invention is not particularly limited, but is preferably 50% to 97%, more preferably 60% to 95%, and even more preferably 70% to 90%. If $\varepsilon_p$ is within the range, it becomes easier to increase the dynamic adsorption capacity even under a high flow rate condition while maintaining the strength of the particle. $\varepsilon_p$ can be determined from the void volume $V_0$ between particles and the total volume of the mobile phase VT, which are obtained in the calculation of the estimated value of the pore diameter, using the following formula.

$$\varepsilon_p = (V_T - V_0)/(V_c - V_0)$$

[In the formula, $V_C$ represents the column volume (mL).]

(2) The pore diameter of the porous particle in a state modified by a ligand is 400 nm or more, preferably 700 nm or more, and more preferably 750 nm or more. The upper limit is preferably 1800 nm or less, more preferably 1500 nm or less, and even more preferably 1200 nm or less.

The pore diameter of the porous particle modified by a ligand can be calculated by a procedure similar to the method for calculating the pore diameter of the porous particle not modified by a ligand, except that a sodium chloride aqueous solution with a concentration of 0.1 mol/L is used instead of pure water as a column filling liquid at the time of compacting the porous particle, and a sodium chloride aqueous solution with a concentration of 1mol/L is used instead of pure water as the mobile phase during retention time measurement, the solvent or dispersion medium of each solution or slurry containing polyethylene glycol with a weight average molecular weight of 106, a measurement sample (standard polyethylene oxide), and excluded particles.

The particle internal porosity $\varepsilon_p$ of the porous particle in the state modified by a ligand is preferably 40% to 95%, more preferably 50% to 90%, and even more preferably 60% to 85%. If $\varepsilon_p$ is within the range, it becomes easier to increase the dynamic adsorption capacity even under a high flow rate condition while maintaining the strength of the particle. $\varepsilon_p$ can be determined from the void volume $V_0$ between particles and the total volume of the mobile phase VT, which are obtained in the calculation of the estimated value of the pore diameter, using the following formula.

$$\varepsilon_p = (V_T - V_0)/(V_c - V_0)$$

[In the formula, $V_C$ represents the column volume (mL).]

The porous particle of the invention is not particularly limited as long as it is a porous particle that satisfies one or more of the properties of (1) to (2), but examples include the following porous particles.

Examples may include: organic porous particles such as synthetic polymer-based porous particles, natural polymer-based porous particles; inorganic porous particles; and combinations thereof, such as organic-organic composite porous particles or organic-inorganic composite porous particles. Examples of natural polymer-based porous particles include polysaccharide-based porous particles (preferably crosslinked polysaccharide-based porous particles) such as cellulose, agarose, dextran, etc. Examples of inorganic porous particles include those formed by glass, silica gel, metals, metal oxides, etc.

Among the above, organic porous particles are preferred, polymer-based porous particles are more preferred, and polysaccharide-based porous particles are even more preferred. Furthermore, among polysaccharide-based porous particles, porous cellulose particles are preferred. The main component of porous cellulose particles is preferably cellulose acetate or cellulose, and it is more preferable to be cellulose acetate or cellulose that has been saponified and crosslinked, as it has no non-specific adsorption of proteins and has sufficient strength to maintain particle shape during liquid flow at a high flow rate.

Here, the main component refers to a component whose content is 50 mass% or more in the porous cellulose particle. In the case where the main component of the porous cellulose particle is cellulose acetate or cellulose, the content of cellulose and/or cellulose acetate in the porous cellulose particles is preferably 60 mass% to 100 mass%, and more preferably 70 mass% to 100 mass%. Also, the degree of acetylation of cellulose acetate is preferably 45% to 57%.

The median diameter of the porous particles of the invention is not particularly limited, but is preferably 40 μm to 200 μm, more preferably 50 μm to 120 μm, and even more preferably 60 μm to 100 μm. If the median diameter is 40 μm or

more, pressure loss can be reduced, and if the median diameter is 200 $\mu$m or less, the surface area of the particles can be increased to bind more biopolymers. The median diameter referred to here can be calculated by measuring the median diameter using, for example, Partica LA-960, which is a laser scattering particle size distribution analyzer manufactured by HORIBA.

The specific surface area of the porous particle of the invention according to the BET multipoint method is preferably 1 m$^2$/g to 200 m$^2$/g. The specific surface area according to the BET multipoint method can be determined by, for example, freezing and drying overnight, pretreating at 100°C for 2 hours by performing vacuum degassing, and then measuring with a high-speed specific surface area/pore distribution measuring apparatus (BELSORP MAXII manufactured by Microtrac BEL), from the adsorption isotherm (straight line) obtained from the measurement of the amount of nitrogen gas adsorbed on the solid surface at a measurement temperature of 77.3K and in a pressure range of three or more points at relative pressures from 0.05 to 0.30.

The method for manufacturing the porous particles of the invention is not particularly limited, but for example, in the case where the porous particles are porous cellulose particles, the porous particles can be manufactured by a method including: (a) a step of preparing a cellulose acetate solution by dissolving cellulose acetate by heating in a mixed solvent of a solvent in which cellulose acetate is soluble and a solvent in which cellulose acetate is insoluble; (b) a step of dispersing the cellulose acetate solution in water containing an emulsion stabilizer to obtain a dispersion system; and (c) a step of cooling the dispersion system to precipitate cellulose acetate particles, where the mixed solvent is an organic solvent that is immiscible with water.

Regarding step (a) of the porous cellulose particle manufacturing process, in step (a), cellulose acetate as the raw material is dissolved in a mixed solvent to prepare a cellulose acetate solution. Cellulose acetate is a semi-synthetic polymer obtained by acetyl esterification of cellulose, which is a natural polymer. The cellulose acetate used in the invention is not particularly limited as long as the cellulose acetate can be generally defined as cellulose acetate, but the cellulose acetate preferably has a degree of polymerization of 50 to 300 and a degree of acetylation of 45 to 57%. By using cellulose acetate with a degree of polymerization of 50 to 300 and a degree of acetylation of 45% to 57%, the cellulose acetate can be dissolved in a wider variety of solvents.

In step (a), the proportion of cellulose acetate in the mixed solvent is preferably 0.1% by mass to 50% by mass, and more preferably 5% by mass to 20% by mass. Within the range, a uniform solution can be obtained, and a porous particle having openings with a uniform diameter can be easily obtained. In addition, precipitation due to phase separation occurs easily upon cooling, resulting in sufficient strength, making it easier to maintain the substantially spherical particle shape. Also, the lower the concentration of cellulose acetate in the cellulose acetate solution, the smaller the particle diameter of the resulting porous particle tends to be, and the larger the pore diameter tends to be. Furthermore, the higher the concentration of cellulose acetate in the cellulose acetate solution, the smaller the particle internal porosity $\varepsilon_p$ tends to be.

In step (a), the solvent in which the cellulose acetate solution is soluble, i.e., a good solvent, is not particularly limited as long as the solution can dissolve cellulose acetate, but an organic solvent with low solubility in water is preferred. "good solvent" as used here refers to a solvent in which the solute dissolves in the solvent alone, resulting in a transparent solution containing no solid matter. In particular, "good solvent" refers to a solvent that can provide a solution with a concentration of preferably 1% by mass or more at a temperature below the boiling point of the solvent. The solvent may be used alone or as a mixture of two or more solvents. Specifically, examples may include benzyl alcohol, ethyl acetate, cyclohexanone, isophorone, and mixtures thereof. Among the above, benzyl alcohol is preferred in terms of creating interconnected pores.

In step (a), the solvent in which the cellulose acetate solution is insoluble, i.e., a poor solvent, includes alcohols, glycols, ethers, esters, and mixtures thereof. A poor solvent refers to a solvent that has no or low solubility for the solute compared to a good solvent, specifically a solvent that cannot dissolve the solute alone at a temperature at or below the boiling point of the solvent and does not provide a transparent solution. In particular, preferably, a poor solvent refers to a solvent that cannot dissolve the solute at a concentration of 1% by mass or more at a temperature equal to or below the boiling point of the solvent. A solvent with low solubility in water is preferred. Especially, the solvent is preferably an alcohol. It is preferable that the alcohol is a lower alcohol, and more preferably 1-hexanol in terms of creating interconnected pores.

In step (a), the volume ratio of the solvent in which cellulose acetate is soluble, i.e., the good solvent, to the solvent in which cellulose acetate is insoluble, i.e., the poor solvent, in the mixed solvent is preferably from 5:95 to 95:5. The higher the ratio of the poor solvent in the mixed solvent, the larger the average diameter of the interconnected pores tends to be.

In step (a), the mixed solvent is an organic solvent that is immiscible with water. If the mixed solvent is to be mixed with water, a dispersion system cannot be attained in step (b).

In step (a), a third component which is a polymer can be included in the mixed solvent. As the polymer as the third component, polyethylene glycol or polypropylene glycol is preferred, and polypropylene glycol is more preferred.

In step (a), when the third component is polypropylene glycol, the molecular weight of polypropylene glycol is not particularly limited, but from the viewpoint of handling during the manufacturing process and the pore diameter exhibited in the porous particles, it is preferable that the average molecular weight (Mw) is 100 to 3000. Also, the higher the molecular weight of the polymer of the third component, the larger the pore diameter tends to be.

In step (a), from the viewpoint of the pore diameter of the porous particles, the concentration of the third component in the mixed solvent is preferably 0.1% by mass to 10% by mass, and the higher the concentration of the third component, the larger the pore diameter tends to be.

In step (a), for the dissolution by heating, the heating temperature is preferably in the range of, for example, 50°C to 130°C. It is preferable that cellulose acetate dissolves in the mixed solvent within such temperature range. The time for such dissolution by heating is not particularly limited, and the heating time can be, for example, 3 to 24 hours.

Regarding step (b) of the porous cellulose particle manufacturing process, the cellulose acetate solution is dispersed in hot water in which an emulsion stabilizer is dissolved. In the step, the volume ratio of the cellulose acetate solution to the hot water is not particularly limited as long as the volume ratio is within a range that allows obtaining a dispersion system where the cellulose acetate solution forms the dispersed phase and the hot water forms the continuous phase. From the viewpoint of obtaining a stable dispersion system, the volume ratio of the cellulose acetate solution to the hot water (dispersed phase/continuous phase) is preferably 1.0 or less. Also, the temperature of the hot water is preferably 50°C to 100°C, and more preferably 60°C to 95°C.

In step (b), regarding the method of dispersion, any known method can be applied as desired. For example, there are methods using a mixer such as a stirrer, methods using a homogenizer, methods using ultrasound, and so on. Also, there are methods of obtaining droplets by extruding the cellulose acetate solution through a thin nozzle by using what is generally called a microreactor, or methods of applying shear by extruding the cellulose acetate solution, or a mixture of the cellulose acetate solution, water, and emulsion stabilizer through a porous membrane with a uniform fine diameter. Among the above, the method of dispersion using a stirrer is preferred because the method is simple.

In step (b), the emulsion stabilizer is not particularly limited as long as the emulsion stabilizer enhances the stability of the dispersion system and has the effect of preventing particle aggregation. Examples may include natural polymers such as starch, pectin, alginic acid, alginate, gelatin; natural polymer processed products such as methyl cellulose, carboxymethyl cellulose, hydroxyethyl cellulose; alcoholic OH-containing synthetic polymers such as polyvinyl alcohol, partially saponified polyvinyl acetate; polymers containing a $SO_3H$ group such as sulfonated styrene; polymers containing a COOH group such as acrylic acid esters; nitrogen-containing synthetic polymers such as polyvinylpyrrolidone; inorganic substance powder such as barium sulfate, talc, bentonite, titanium oxide; anionic surfactants such as sodium linear alkylbenzene sulfonate; cationic surfactants such as alkyltrimethylammonium salts; amphoteric surfactants such as alkyldimethylamine oxide; nonionic surfactants such as fatty acid sorbitan esters; or mixtures of thereof. Particularly, from the viewpoint of stabilizing the dispersion system, sodium dodecylbenzene sulfonate, polyvinyl alcohol, carboxymethyl cellulose, hydroxyethyl cellulose, or mixtures thereof are preferred.

In step (b), the addition amount of emulsion stabilizer is preferably 0.01 mass% or more as the concentration in hot water from the viewpoint of stabilizing the dispersion system. In such concentration range, when the addition amount of emulsion stabilizer is increased, the particle diameter of the obtained porous cellulose particles tends to become smaller. Conversely, when the amount of addition of the emulsion stabilizer is decreased, the particle diameter of the obtained porous cellulose particles tends to become larger.

In step (c) of the porous cellulose particle manufacturing process, the dispersion liquid obtained in step (b) is cooled. By cooling, thermally induced phase separation occurs, and porous cellulose particles having an interconnected pore structure can be obtained.

In step (c), the temperature for cooling the dispersion system is not particularly limited as long as it is a temperature at which cellulose acetate precipitates. However, from the viewpoint that porous cellulose particles can sufficiently precipitate, the cooling temperature is preferably 0°C to 50°C.

In step (c), the cooling rate of the dispersion system is preferably 0.1°C/min to 10°C/min, more preferably 0.1°C/min to 5°C/min, and even more preferably 0.1°C/min to 2°C/min. By adjusting the cooling rate, particles with pore diameters suitable for the intended purpose can be obtained.

By the manufacturing method, porous cellulose particles using cellulose acetate as a raw material can be obtained. In the case of imparting hydrophilicity to the porous cellulose particles by using cellulose acetate as a raw material, the cellulose acetate can be converted to cellulose by saponification in a solution of an alkali metal hydroxide compound such as sodium hydroxide.

The porous particles of the invention may be further saponified and crosslinked. Saponification and crosslinking processes can be performed by conventional methods, and for example, the crosslinking process of porous cellulose particles can be performed with reference to Japanese Patent Application Laid-Open Publication No. 2009-242770. As the crosslinking agent, any compound having two or more functional groups capable of reacting and binding with the hydroxyl group of cellulose can be used as the crosslinking agent, for example, halohydrins such as epichlorohydrin, epibromohydrin, dichlorohydrin; bifunctional bisepoxides (bisoxiranes); multifunctional polyepoxides (polyoxiranes) such as glycerol polyglycidyl ether.

The porous particle of the invention may be modified with a ligand. That is, at least some of the reactive functional groups of the porous particle of the invention may be modified with a ligand. Ligands are compounds that directly bind to the surface of the porous particles, or bind via an epoxy group, a formyl group, a vinyl group, etc., introduced on the particle surface, and is not particularly limited as long as the ligand has affinity with the adsorption target. Examples may include ligands containing a conventional ion exchange group, affinity ligands, ligands containing a hydrophobic group, ligands containing a charged group, etc. The ligands may be introduced alone or in appropriate combinations of multiple ligands.

As the ion exchange group, examples may include a 2-diethylaminoethyl (DEAE) group, a carboxymethyl (CM) group, a sulfonic acid group, a quaternary ammonium (Q) group, etc.

As the hydrophobic group, examples may include a phenyl group, an n-butyl group, an n-hexyl group, an n-octyl group, an n-octadecyl group, etc.

Also, conventional ligands that possess both an ion exchange group and a hydrophobic group, which can be used for the so-called mixed-mode separation, may be used.

As the affinity ligands, examples may include ligands containing a sulfate ester group (e.g., sulfated polysaccharides), protein adsorption ligands such as Protein A, an antibody, etc., polycations such as polylysine, functional polymers such as heparin, polyglutamic acid, polyanion containing a phosphate ester group, etc.

Porous particles modified with any ligand can be used according to the purpose of purification. As the ligands, one or more selected from the group consisting of ligands containing an ion exchange group, affinity ligands, and ligands containing a hydrophobic group are preferable.

In the case where the porous particle of the invention is modified with a ligand containing a sulfur element, such as ligands containing a sulfonic acid group or ligands containing a sulfate ester group, the sulfur content of the porous particle is preferably 0.1 weight% to 10.0 weight%, and more preferably 1.0 weight% to 5.0 weight%.

The sulfur content is an indicator of the modification amount of the ligand containing a sulfur element, and a higher sulfur content indicates a larger modification amount of the ligand containing a sulfur element. The sulfur content can be calculated, for example, by drying the ligand-modified porous particle and determining the sulfur content (weight%) per dry weight through a carbon hydrogen nitrogen sulfur (CHNS) elemental analysis using an automatic elemental analyzer, i.e., vario EL cube (manufactured by Elementar).

The sulfur content can be adjusted by the addition amount of the compound having the ligand (2-acrylamido-2-methylpropanesulfonic acid, dextran sulfate sodium, etc.), and may be appropriately determined according to the purpose of purification, etc.

The ligand can be introduced by conventional methods, and the ligand-modified porous particle can be suitably used as a carrier for various chromatography such as affinity chromatography, ion exchange chromatography, chelate chromatography, hydrophobic interaction chromatography, etc. The porous particles of the invention are suitable for separation and purification of biopolymers as the target substance due to the size of the pore diameter, and the chromatography carrier with introduced affinity ligands, charged groups, hydrophobic groups, etc., can be preferably used for the purification of biopolymers.

The size of the biopolymer to be purified is preferably 1 nm to 100 nm, more preferably 1 nm to 25 nm, and even more preferably 5 nm to 10 nm. Within the range, a biopolymer can easily enter the pores of the porous particle of the invention, and it becomes easier to maintain a high adsorption capacity even under a high flow rate condition.

Examples of biopolymers include polypeptides, such as polypeptides isolated by conventional methods after being obtained by expression in Escherichia coli, yeast, or animal cells, proteins, synthetic products such as synthetic polypeptides or synthetic proteins, and nucleic acids (e.g., DNA, etc.), as well as the derivatives thereof (e.g., glycoproteins, DNA conjugates), viruses (e.g., adeno-associated viruses, influenza viruses), etc. Among the above, polypeptides, proteins and the derivatives thereof are preferable as biopolymers, and proteins and the derivatives thereof, especially antibodies, are more preferable.

The liquid chromatography carrier of the invention includes the porous particle of the invention.

Also, the liquid chromatography apparatus of the invention includes a separation column filled with the liquid chromatography carrier of the invention.

The separation column is the same as a normal liquid chromatography separation column except that it is filled with the liquid chromatography carrier of the invention. Specifically, the separation column includes a column container and the liquid chromatography carrier of the invention filled in the column container.

The method for filling the liquid chromatography carrier of the invention into the separation column is not particularly limited, but can be performed, for example, by flow packing, dynamic axial compression, or pack-in-place.

For the liquid chromatography apparatus of the invention, a conventional liquid chromatography apparatus can be used except that the liquid chromatography apparatus includes a separation column filled with the liquid chromatography carrier of the invention. The liquid chromatography apparatus of the invention may form a portion of various analytical devices such as HPLC, LC/MS, or LC/MS/MS. The liquid chromatography apparatus has, for example, an autosampler as a sample solution introduction part, a pumping device that delivers the sample solution to the separation column, and a detection part that optically detects the separated sample in the separation column. The detection results at the detection part are subjected to necessary analytical processing as digital information in a data processing device within the liquid chromatograph, and are output to an output device such as a printer or a display device. Alternatively, the results may be output to another data processing device or data storage medium.

The separation and purification method of the biopolymer of the invention includes a step of separating and purifying the biopolymer by using the liquid chromatography apparatus of the invention. According to the separation and purification method of the biopolymer of the invention, even under a high flow rate condition, for example, a condition in which the column residence time of the mobile phase containing the biopolymer is as short as 20 seconds or less, 10 seconds or less, 7.5 seconds or less, the productivity of the target biopolymer bound per unit volume, per unit time can be improved. The "column residence time" means a value obtained by dividing "column volume" by "flow rate when the mobile phase containing the biopolymer is injected into the separation column".

The description regarding the biopolymer incorporates the above description by reference.

[Examples]

The invention will be described more specifically by the following examples, but the invention is not limited to the embodiments of these examples.

Particles of Manufacturing Examples 1 to 7 were prepared according to the following procedure. Manufacturing Examples 1 to 3 and 7 correspond to Examples, and Manufacturing Examples 4 to 6 correspond to Comparative Examples.

[Manufacturing Example 1]

· Granulation process

(1) 100g of cellulose acetate (L-20, manufactured by Daicel Corporation) was added to a mixed solvent of 391 g of benzyl alcohol, 276g of 1-hexanol, and 14g of polypropylene glycol (Wako Pure Chemical reagent, diol type, average molecular weight 1,000) and stirred.

(2) The mixture was heated and stirred at 120°C for 4 hours to dissolve the cellulose acetate, and a transparent cellulose acetate solution was obtained.

(3) 2g of PVA (JP-18E, manufactured by JAPAN VAM & POVAL CO., LTD.) and 30g of sodium carboxymethyl cellulose (CMC1140, manufactured by Daicel Miraizu Corporation) were added to 2300g of pure water saturated with a mixed solvent, heated and stirred at 80°C for 1 hour or more to dissolve, thereby obtaining a dispersion medium.

(4) 770g of the cellulose acetate solution was quickly poured into 2300g of the dispersion medium, and stirred at 80°C to obtain a dispersion system. Subsequently, the dispersion system was cooled to 35°C to obtain spherical cellulose acetate particles.

(5) The obtained cellulose acetate particles were thoroughly washed with a large amount of water, then with methanol, and then washed again with pure water.

· Saponification process

(1) 630 g (wet) of the obtained cellulose acetate particles (water content ratio: 6.86) were added to a mixture of 1072g of pure water and 242g of methanol, and stirred for 30 minutes at a temperature of 35°C.

(2) 201g of 20% NaOH was added and stirred at 35°C for 2 hours to perform saponification by reaction.

(3) After cooling to 30°C or below and neutralizing with acetic acid, the mixture was thoroughly washed with pure water, and the obtained saponified cellulose spherical particles were passed through sieves with openings of 106 $\mu$m and 32 $\mu$m to obtain saponified cellulose particles with particle diameters of 32 $\mu$m to 106 $\mu$m.

· Crosslinking process

(1) 96.4 g (wet) of the obtained saponified cellulose particles (water content ratio 7.93) were dispersed in 194 g of pure water, and then 78.6 g of $Na_2SO_4$ was dissolved.

(2) After being stirred at a temperature of 50°C for 30 minutes, 4.0 g of 48% NaOH and 0.69 g of $NaBH_4$ were added, dissolved, and reacted over 30 minutes.

(3) After dissolution, 36.4 mL of 48% NaOH solution and 62 g of epichlorohydrin, each divided into 8 equal portions, were added over approximately 4 hours at 30-minute intervals.

(4) After completion of the addition, the reaction was performed at a temperature of 50°C for 16 hours. The temperature was cooled to 30°C or below, and acetic acid was added for neutralization.

(5) The reaction mixture was filtered to recover the gel, then filtered and washed by pure water to obtain crosslinked porous particles.

[Manufacturing Example 2]

(1) 681 g of cellulose acetate (L-20, manufactured by Daicel Corporation) was added to a mixed solvent of 2671 g of benzyl alcohol, 1883 g of 1-hexanol, and 97 g of polypropylene glycol (Wako Pure Chemical reagent, diol type, average molecular weight 1,000) and stirred.

(2) The mixture was heated and stirred at 120°C for 4 hours to dissolve the cellulose acetate, and a transparent cellulose acetate solution was obtained.

(3) 14g of PVA (JP-18E, manufactured by JAPAN VAM & POVAL CO.,LTD.) and 252 g of sodium carboxymethyl cellulose (CMC1140, manufactured by Daicel Miraizu Corporation) were added to 18 kg of pure water saturated with a mixed solvent, heated and stirred at 80°C for 1 hour or more to dissolve, thereby obtaining a dispersion medium.

(4) 5300 g of the cellulose acetate solution was poured into 16 kg of the dispersion medium, and stirred at 80°C to obtain a dispersion system. Subsequently, the dispersion system was cooled from 80°C to 35°C in approximately one hour to obtain spherical cellulose acetate particles.

(5) The obtained cellulose acetate particles were thoroughly washed with a large amount of water, then with methanol, and then washed again with pure water. The obtained cellulose acetate particles were saponified according to the same procedure as in Manufacturing Example 1, and the obtained saponified cellulose spherical particles were passed through sieves with openings of 150 $\mu$m and 45 $\mu$m to obtain saponified cellulose particles with particle diameters of 45 $\mu$m to 150 $\mu$m. Subsequently, crosslinking was performed according to the same procedure as in Manufacturing Example 1 to obtain crosslinked porous particles.

[Manufacturing Example 3]

A dispersion system obtained by mixing and stirring the cellulose acetate and the dispersion medium prepared in the same manner as in Manufacturing Example 2 was cooled from 80°C to 35°C over approximately 50 minutes to obtain spherical cellulose acetate particles.

The obtained cellulose acetate particles were saponified, classified, and crosslinked according to the same procedure as in Manufacturing Example 2 to obtain crosslinked porous particles.

[Manufacturing Example 4]

A dispersion system obtained by mixing and stirring the cellulose acetate and the dispersion medium prepared in the same manner as in Manufacturing Example 2 was cooled from 80°C to 35°C over approximately 90 minutes to obtain spherical cellulose acetate particles.

The obtained cellulose acetate particles were saponified, classified, and crosslinked according to the same procedure as in Manufacturing Example 2 to obtain crosslinked porous particles.

[Manufacturing Example 5]

100 g of cellulose acetate (L-20, manufactured by Daicel Corporation) was added to a mixed solvent of 391 g of benzyl alcohol, 276 g of 1-hexanol, and 7 g of polypropylene glycol (Wako Pure Chemical reagent, diol type, average molecular weight 1,000) and stirred. Subsequently, spherical cellulose acetate particles were obtained according to the same procedure as in Manufacturing Example 1.

The obtained cellulose acetate particles were saponified, classified, and crosslinked according to the same procedure as in Manufacturing Example 1 to obtain crosslinked porous particles.

[Manufacturing Example 6]

· Granulation process

(1) 6.4g of crystalline cellulose (manufactured by Asahi Kasei Chemicals Corporation, product name: Ceolus PH101) was added to 100g of an aqueous solution of 60 weight% calcium thiocyanate, and dissolved by heating to 110°C to 120°C.

(2) 6g of sorbitan monooleate was added as a surfactant to the solution, and the mixture was dripped into 480m L of o-dichlorobenzene preheated to 130-140°C and stirred and dispersed.

(3) The dispersion was cooled to 40°C or below, poured into 190mL of methanol, and a suspension of particles was obtained.

(4) The suspension was filtered and separated, the particles were washed with 190mL of methanol, and filtered and separated. The washing operation was performed several times.

(5) After washing with a large amount of water, spherical cellulose particles were obtained.

(6) The spherical cellulose particles were passed through sieves with openings of 125$\mu$m and 53$\mu$m, and cellulose particles with particle diameters of 53$\mu$m to 125$\mu$m were obtained.

· Crosslinking process

(1) 121g of pure water was added to 100g of the obtained cellulose particles, and the mixture was heated while being stirred. When the temperature reached 30°C, 3.3g of 45 weight% NaOH aqueous solution and 0.5g of NaBH$_4$ were added and stirred. The initial alkali concentration was 0.69% (w/w).

(2) After 30 minutes, 60g of Na$_2$SO$_4$ was added to and disolved in the reaction solution. When the temperature of the mixture reached 50°C, stirring continued for 2 hours.

(3) While continuing to stir the mixture at 50°C, 48g of 45 weight% NaOH aqueous solution and 50g of epichlorohydrin were each divided into 25 equal portions and added over approximately 6 hours at 15-minute intervals.

(4) After completion of the addition, the mixture was reacted at the temperature of 50°C for 16 hours.

(5) After the mixture was cooled to the temperature of 40°C or below, 2.6g of acetic acid was added and neutralized.

(6) The reaction mixture was filtered to recover the gel, then filtered and washed by pure water to obtain crosslinked cellulose particles.

[Manufacturing Example 7]

(1) 28 g of cellulose acetate (L-20, manufactured by Daicel Corporation) was added to a mixed solvent of 110 g of benzyl alcohol, 77 g of 1-hexanol, and 4 g of polypropylene glycol (Wako Pure Chemical reagent, diol type, average molecular weight 1,000) and stirred.

(2) The mixture was heated and stirred at 120°C for 4 hours to dissolve the cellulose acetate, and a transparent cellulose acetate solution was obtained.

(3) 0.46 g of PVA (JP-18E, manufactured by JAPAN VAM & POVAL CO.,LTD.) and 6.8 g of sodium carboxymethyl cellulose (CMC 1140, manufactured by Daicel Miraizu Corporation) were added to 570g of pure water saturated with a mixed solvent, heated and stirred at 80°C for 1 hour or more to dissolve, thereby obtaining a dispersion medium.

(4) 219g of the cellulose acetate solution was poured into 577 g of the dispersion medium, and stirred at 80°C to obtain a dispersion system. Subsequently, the dispersion system was cooled from 80°C to 35°C in approximately one hour to obtain spherical cellulose acetate particles.

(5) The obtained cellulose acetate particles were thoroughly washed with a large amount of water, then with methanol, and then washed again with pure water. The obtained cellulose acetate particles were saponified according to the same procedure as in Manufacturing Example 1, and the obtained saponified cellulose spherical particles were passed through sieves with openings of 150 $\mu$m and 45 $\mu$m to obtain saponified cellulose particles with particle diameters of 45 $\mu$m to 150 $\mu$m. Subsequently, crosslinking was performed according to the same procedure as in Manufacturing Example 1 to obtain crosslinked porous particles.

[Example 1]

· Ligand Modification Process

21g of 2-acrylamido-2-methylpropanesulfonic acid was dissolved in 40g of pure water, then 8.4g of 48.7% (w/w) sodium hydroxide aqueous solution was added for neutralization, and further 20g (wet) of porous particles obtained by Manufacturing Example 1 was added to form a slurry. After depressurizing the reaction vessel and filling the nitrogen gas 3 times, while stirring under nitrogen atmosphere, a solution prepared by dissolving 0.9g of ammonium cerium nitrate in 9.5 mL of 0.17 mol/mL nitric acid aqueous solution was slowly added to the reaction vessel. After the addition, the reaction vessel was heated to 40°C while maintaining the nitrogen atmosphere, and the reaction was carried out for 16 hours. The reaction mixture was filtered to recover the gel, and was washed 3 times with pure water. Next, the product was washed 10 times with 80 mL of 1mol/L sulfuric acid, then washed with pure water until the washing liquid became neutral. Subsequently, it was washed with 80 mL of 0.5mol/L sodium hydroxide, and finally filtered and washed with pure water until the washing liquid became neutral, to obtain ligand-modified porous particles.

[Example 2]

Except for using the crosslinked porous particles obtained in Manufacturing Example 2, the ligand modification reaction was performed following the same procedure as in Example 1 to obtain ligand-modified porous particles.

[Example 3]

Except for using the crosslinked porous particles obtained in Manufacturing Example 3, the ligand modification reaction was performed following the same procedure as in Example 1 to obtain ligand-modified porous particles.

[Example 4]

· Ligand Modification Process

(Epoxidation Step)

(1) 60 g (wet) of crosslinked porous particles obtained from Manufacturing Example 7 (water content rate 5.01) were added to 103 mL of pure water and stirred.
(2) The internal temperature was adjusted to 30°C, and 58.5 g of epichlorohydrin was added.
(3) After stirring for 15 minutes, 54.7 g of 48% NaOH aqueous solution was added and the reaction was performed for 2 hours.
(4) After the reaction was completed, the neutral was reached by using acetic acid, then the solution was thoroughly washed with water until the filtrate became neutral, and epoxy active porous particles were obtained.
(5) 1.0 g of epoxy active porous particles was oscillated for 1 hour with a 3.0 mL of 1.3 M sodium thiosulfate solution by using an oscillator set at 30°C for 1 hour, and the epoxy amount was measured by titration with 0.1 mol/L hydrochloric acid. The epoxy group basic amount was 229 $\mu$mol/g (dry).

(Binding Step of Sulfated Polysaccharide)

(1) 4.0 g of dextran sulfate sodium DS-500 (manufactured by Meito Sangyo Co., Ltd.) was added to 51 mL of pure water and stirred until the dextran sulfate sodium was completely dissolved.
(2) 30 g (wet) of epoxy active porous particles was added to the reactor.
(3) While stirring thoroughly, the internal temperature was adjusted to 30°C, and after stirring for 15 minutes, 20.7 g of sodium sulfate, 22.1 g of disodium hydrogen phosphate, and 3.2 g of 48% NaOH aqueous solution were added.
(4) The temperature was raised to 40°C, and the reaction was performed for 6 hours.
(5) After the reaction was completed, a solution prepared by dissolving 3.3 mL of 48% NaOH aqueous solution and 0.5 g of sodium tetrahydroborate in 23.8 mL of pure water was added, and the reaction was continued for another 16 hours.
(6) After the reaction, the reaction liquid was filtered and thoroughly washed with water until the filtrate became neutral to obtain dextran sulfated porous particles.

[Comparative Example 1]

Except for using the crosslinked porous particles obtained in Manufacturing Example 4, the ligand modification reaction was performed following the same procedure as in Example 1 to obtain ligand-modified porous particles.

[Comparative Example 2]

Except for using the crosslinked porous particles obtained in Manufacturing Example 5, the ligand modification reaction was performed following the same procedure as in Example 1 to obtain ligand-modified porous particles.

[Comparative Example 3]

· Ligand Modification Process

8.7g of 2-acrylamido-2-methylpropanesulfonic acid was dissolved in 64.4 g of pure water, then 3.4g of 48.7% (w/w) sodium hydroxide aqueous solution was added for neutralization, and further 80g (wet) of porous particles obtained by Manufacturing Example 6 was added to form a slurry. After depressurizing the reaction vessel and filling the nitrogen gas 3 times, while stirring under nitrogen atmosphere, a solution prepared by dissolving 5.19g of ammonium cerium nitrate in 18.1 mL of 0.17 mol/mL nitric acid aqueous solution was slowly added to the reaction vessel. After the addition, the reaction vessel was heated to 40°C while maintaining the nitrogen atmosphere, and the reaction was carried out for 16 hours. The reaction mixture was filtered to recover the gel, and was washed 3 times with pure water. Next, the product was washed 10 times with 80 mL of 1mol/L sulfuric acid, then washed with pure water until the washing liquid became neutral. Subsequently, it was washed with 80 mL of 0.5mol/L sodium hydroxide, and finally filtered and washed with pure water until the washing liquid became neutral, to obtain ligand-modified porous particles.

[Comparative Example 4]

As the ligand-modified porous particles, POROS XS strong cation exchange resin purchased from Thermo Fisher Scientific Inc. was used.

[Comparative Example 5]

As the ligand-modified porous particles, POROS 50HS strong cation exchange resin purchased from Thermo Fisher Scientific Inc. was used.

[Comparative Example 6]

· Ligand Modification Process

(Epoxidation Step)

(1) 40 g (wet) of crosslinked porous particles obtained from Manufacturing Example 6 were added to 30 mL of pure water and stirred.
(2) The internal temperature was adjusted to 30°C, and 24.7 g of epichlorohydrin was added.
(3) After stirring for 15 minutes, 23.1 g of 48% NaOH aqueous solution was added and the reaction was performed for 2 hours.
(4) After the reaction was completed, the neutral was reached by using acetic acid, then the solution was thoroughly washed with water until the filtrate became neutral, and epoxy active porous particles were obtained.
(5) 1.0 g of epoxy active porous particles was oscillated for 1 hour with a 3.0 mL of 1.3 M sodium thiosulfate solution by using an oscillator set at 30°C for 1 hour, and the epoxy amount was measured by titration with 0.1 mol/L hydrochloric acid. The epoxy group amount was 230 $\mu$mol/g (dry).

(Binding Step of Sulfated Polysaccharide)

(1) 3.3 g of dextran sulfate sodium DS-500 (manufactured by Meito Sangyo Co., Ltd.) was added to 25.6 mL of pure water and stirred until the dextran sulfate sodium was completely dissolved.
(2) 40 g (wet) of epoxy active porous particles was added to the reactor.
(3) While stirring thoroughly, the internal temperature was adjusted to 30°C, and after stirring for 15 minutes, 16.7 g of sodium sulfate, 26.8 g of disodium hydrogen phosphate, and 2.6 g of 48% NaOH aqueous solution were added.

(4) The temperature was raised to 40°C, and the reaction was performed for 6 hours.

(5) After the reaction was completed, a solution prepared by dissolving 3.1 mL of 48% NaOH aqueous solution and 0.2 g of sodium tetrahydroborate in 28.3 mL of pure water was added, and the reaction was continued for another 16 hours.

(6) After the reaction, the reaction liquid was filtered and thoroughly washed with water until the filtrate became neutral to obtain dextran sulfated porous particles.

[Measurement Method 1: Measurement of Median Diameter]

For the particles obtained in Manufacturing Examples 1 to 7, Examples 1 to 4, and Comparative Examples 1 to 6, the particle size distribution was measured and the median diameter was calculated. The apparatus used for measurement is as follows.

Apparatus: Laser Scattering Particle Size Distribution Analyzer Partica LA-960 (manufactured by HORIBA)
The median diameter was measured by using such apparatus.

[Measurement Method 2: Measurement of Retention Time of Crosslinked Particles, Estimation of Pore Diameter, Estimation of Particle Internal Porosity]

For the particles obtained in Manufacturing Examples 1-7, after filling the particles into a stainless steel column (manufactured by Tosoh) with an inner diameter of 0.78 cm and a length of 30 cm, the retention time of each sample shown in Table 1 was measured. In the filling method, the particles were dispersed in pure water to make a slurry, the slurry was then filled into the column, and then pure water flew at a flow rate of 0.4 mL/min for 1 hour or more to compact the particles. Pure water was used as the dispersion medium for each sample and as the mobile phase at the time of measuring the retention time of each sample. In addition, the column temperature at the time of measurement was adjusted to 25°C. The value at which the RI detection intensity reached the maximum was taken as the elution peak, and such time was taken as the retention time. The 1260 Infinity HPLC apparatus (manufactured by Agilent Technologies) was used as the measuring apparatus.

[Table 1]

[Table 1]

| Sample | Weight average molecular weight $M_w$ (Da) | Viscosity radius $r_s$ (nm) |
|---|---|---|
| Polyethylene oxide SE-150 (by Tosoh) | $7.86 \times 10^5$ | 40.14 |
| Polyethylene oxide SE-70 (by Tosoh) | $5.80 \times 10^5$ | 33.12 |
| Polyethylene oxide SE-30 (by Tosoh) | $2.80 \times 10^5$ | 22.43 |
| Polyethylene oxide SE-15 (by Tosoh) | $1.49 \times 10^5$ | 15.35 |
| Polyethylene oxide SE-8 (by Tosoh) | $1.07 \times 10^5$ | 12.01 |
| Polyethylene oxide SE-5 (by Tosoh) | $3.76 \times 10^4$ | 7.2 |
| Polyethylene oxide SE-2 (by Tosoh) | $2.36 \times 10^4$ | 5.11 |
| PEG106 (by Agilent Technologies) | 106 | 0.33 |
| Silica fine particles with particle diameter of 100 nm (by micromod, sicaster, Product no.: 43-00-102) | - | - |

The sample concentration was 5 mg/mL, and 10 $\mu$l of each sample was used for the measurement.

The pore diameter of the particles was calculated according to the following procedure.

(i) The total volume $V_T$ (mL) of the mobile phase was calculated by the formula "retention time (min) of polyethylene glycol (PEG106) with a weight average molecular weight of 106 × flow rate (mL/min) × column volume (mL)". The flow rate was 0.4 mL/min and the column volume was 14.34 mL for calculating $V_T$.

(ii) The void volume $V_0$ (mL) between particles was calculated by the formula "excluded particle retention time (min) × flow rate (mL/min) × column volume (mL)". The flow rate was 0.4 mL/min and the column volume was 14.34 mL for calculating $V_0$. The value of the excluded particle retention time was fixed at 13.6 minutes (i.e., the average of the retention time of silica fine particles in Manufacturing Examples 4 to 6) for all of the manufacturing examples to calculate $V_0$.

(iii) The elution volume $V_R$ (mL) of the measurement sample (standard polyethylene oxide) was calculated by the formula "retention time (min) of the measurement sample × flow rate (mL/min) × column volume (mL)". The flow rate was 0.4 mL/min and the column volume was 14.34 mL for calculating $V_R$.

The distribution coefficient $K_d$ was calculated by the following formula.

$$K_d=(V_R\text{-}V_0)/(V_T\text{-}V_0)$$

(v) The viscosity radius $r_s$ (nm) of the measurement sample was calculated based on the weight average molecular weight $M_w$ (Da) and intrinsic viscosity $[\eta]$ (mL/g) of each measurement sample, using the following equation:

$$r_s=(3[\eta]M_w/10_\pi N)^{1/3}\times10^7$$

[In the formula, N represents Avogadro's number.]

(vi) Assuming a single pore diameter, the distribution coefficient $K_d$ and the hydraulic radius $r_{hyd}$ of the particle are expressed by the following formula.

$$K_d=(1\text{-}r_s/r_{hyd})^2$$

Like the samples shown in Table 1, the retention times for the 7 samples from PEG-21300 to PEG-400 shown in Table 2 were also measured. However, the measurement results of the 7 samples shown in Table 2 were not used for estimating the pore diameter for the following reasons. That is, by taking 0.5 power of K when each measurement sample was measured as the vertical axis and the viscosity radius $r_s$ of each measurement sample as the horizontal axis and obtaining $-1/r_{hyd}$ from the slope during linear approximation based on $K_d^{0.5}=1\text{-}r_s/r_{hyd}$, there was a distribution of pore diameters in actual porous particles, and a significant difference in slope was present between the region where the viscosity radius of the measurement sample was 5 nm or less (when the measurement sample is PEG21300 to PEG400) and the case where the viscosity radius was greater than 5 nm (when the measurement sample is SE-150 to SE-2). Therefore, in order to calculate the pore diameter of large pores that affect the adsorption of biopolymers, the 0.5 power of $K_d$ obtained by measuring polyethylene oxides of the seven samples from SE-150 to SE-2 with a viscosity radius of 5 nm or more was taken as the vertical axis, and the viscosity radius $r_s$ of each sample was taken as the horizontal axis, and $-1/r_{hyd}$ was obtained from the slope during linear approximation based on the following formula.

$$K_d^{0.5}=C\text{-}r_s/r_{hyd}$$

[In the formula, C represents the intercept during linear approximation.]

(vii) Assuming that the pores of the particle are cylindrical through-holes, the pore diameter $r_{pore}$ and the hydraulic radius $r_{hyd}$ of the particle are represented by the following formula.

$$r_{pore}=r_{hyd}\times2$$

From the formula, the pore diameter $r_{pore}$ of the particle was calculated as an estimated value of the pore diameter of the particle.

The particle internal porosity $\varepsilon_p$ of the particles was calculated from the void volume $V_0$ between particles and the total volume of the mobile phase $V_T$, which were obtained in the calculation of the estimated value of the pore diameter, using the following formula.

$$\varepsilon_p=(V_T\text{-}V_0)/(V_c\text{-}V_0)$$

The column volume $V_c$ was calculated as 14.34 mL.

[Measurement Method 3: Measurement of Retention Time of Ligand-Modified Particles, Estimation of Pore Diameter, Estimation of Particle Internal Porosity]

For the particles obtained in Examples 1 to 4 and Comparative Examples 1 to 6, after filling the particles into a stainless steel column (manufactured by Tosoh) with an inner diameter of 0.78 cm and a length of 30 cm, the retention time of each sample was measured. The measurement was performed in the same procedure as measuring the retention time for the particles of Manufacturing Examples 1-7, except that the column filling liquid at the time of compaction was a 0.1 mol/L sodium chloride aqueous solution, and the dispersion medium or solvent for each sample and the mobile phase during retention time measurement was a 1 mol/L sodium chloride aqueous solution. The retention time was measured by using the same samples as in Table 1, except that silica nanoparticles were not used for measurement as the silica nanoparticles aggregate and precipitate in the sodium chloride aqueous solution. In addition, the pore diameter of the particles was calculated using the same procedure as the estimation of the pore diameter of the crosslinked particles. However, since the pore diameter of the particles of Comparative Example 3 was small, the molecular weight dependence of the retention time of polyethylene oxide with a molecular weight from 580,000 to 786,000 was small, and there was concern about overestimating the pore diameter, so the pore diameter was calculated by using the $K_d$ of 5 samples from SE-30 to SE-2. Furthermore, for the particles of Comparative Example 6, the retention times of SE-150 to SE-5 were all approximately 12.4 minutes, which was lower than the assumed value of 13.6 minutes for the excluded particle retention time. Therefore, only for Comparative Example 6, the excluded particle retention time was set to 12.4 minutes to estimate the pore diameter. Also, as the sample for calculating the pore diameter of Comparative Example 6, $K_d$ at the time of measuring 7 samples from PEG-21300 to PEG-400 shown in Table 2 was used to estimate the pore diameter. The particle internal porosity was also calculated by using the same procedure as that for estimating the particle internal porosity of the crosslinked particles.

[Table 2]

[Table 2]

| Sample | Weight average molecular weight $M_w$ (Da) | Viscosity radius $r_s$ (nm) |
|---|---|---|
| PEG21300(Agilent Technologies) | 20440 | 4.81 |
| PEG16100(Agilent Technologies) | 15260 | 4.15 |
| PEG7830(Agilent Technologies) | 7460 | 2.87 |
| PEG4040(Agilent Technologies) | 4110 | 2.03 |
| PEG101(Agilent Technologies) | 1020 | 0.98 |
| PEG610(Agilent Technologies) | 620 | 0.75 |
| PEG400(Agilent Technologies) | 400 | 0.62 |
| PEG106(Agilent Technologies) | 106 | - |

The sample concentration was 5 mg/mL, and 10 $\mu$l of each sample was used for the measurement.

[Measurement Method 4: Measurement of Sulfur Content of Ligand-Added Particles]

The particles of Examples 1 to 4 and Comparative Examples 1 to 6 were dried, and the sulfur content (weight%) per dry weight was determined by performing carbon hydrogen nitrogen sulfur (CHNS) elemental analysis. The apparatus used for measurement is as follows.

Apparatus: Fully automatic elemental analyzer vario EL cube (manufactured by Elementar)
The sulfur content was measured by using the apparatus.

[Measurement Method 5: Measurement of 10% Dynamic Binding Capacity (DBC) Using Polyclonal Antibody and Calculation of 10% Dynamic Binding Capacity (DBC) per Unit Time, per Unit Volume]

(1) Apparatus and reagents used:

LC system: AKTA avant 25 (registered trademark)
Buffer: Acetate buffer pH 5.0 (containing 0.05mol/L NaCl)
Polyclonal antibody: $\gamma$-globulin, derived from human serum (Wako Pure Chemical)
Column: 6.7 mm in diameter, 30 mm in length

(2) Measurement method:

First, the polyclonal antibody was dissolved in a buffer to prepare an antibody solution of 2 mg/mL. Then, each column was filled with the ligand-modified particles (particles from Manufacturing Examples 1-4, Comparative Examples 1-6) without gaps. Next, the column was connected to the LC system and the buffer was flowed until the UV (ultraviolet absorbance, 280nm), electrical conductivity, and pH of the column effluent became constant for equilibration. After that, the baseline UV was set to zero. Then, the antibody solution was flowed through the column at a flow rate of 8.48 mL/min (column residence time of 7.5 seconds). The UV of the column effluent was monitored, and the time during which the antibody solution was flowed until the UV of the column effluent reached 10% of the UV of the antibody solution, which had been measured in advance, was read. The 10% dynamic binding capacity of the antibody was calculated by using the following formula. The analysis was performed in a room at 25°C.

{Antibody solution concentration (mg/mL) $\times$ Time (min) from the start of flowing the antibody solution until reaching 10% of the UV of the antibody solution measured in advance $\times$ Flow rate (mL/min) - Dead volume}/Column volume = 10% dynamic binding capacity (mg/mL) [In the formula, the dead volume is the volume (mL) obtained by adding the system piping volume and the column void volume.]
The dead volume was determined by flowing a 1 mol/L sodium chloride solution through the column for equilibration until the UV (ultraviolet absorbance, 280nm), electrical conductivity, and pH of the column effluent became constant, then injecting an amount of 2mg/mL antibody solution equivalent to approximately 1% of the column volume, and then flowing the 1mol/L sodium chloride solution again and measuring the flow rate of the sodium chloride solution until a UV peak was detected.
In addition, by using the value of the 10% dynamic binding capacity (mg/mL) calculated from the above formula, the 10% dynamic binding capacity of the antibody per unit time and per unit volume (mg/mL/min) under the condition of column residence time of 7.5 seconds was determined using the following formula.

10% dynamic binding capacity (mg/mL)/Column volume (mL)/0.125 (min) = 10% dynamic binding capacity per unit time and per unit volume (mg/mL/min).

[Measurement Method 6: SEM Observation]

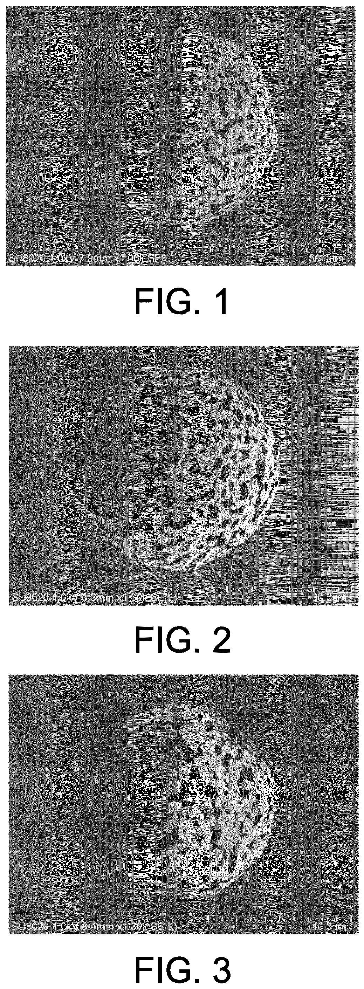

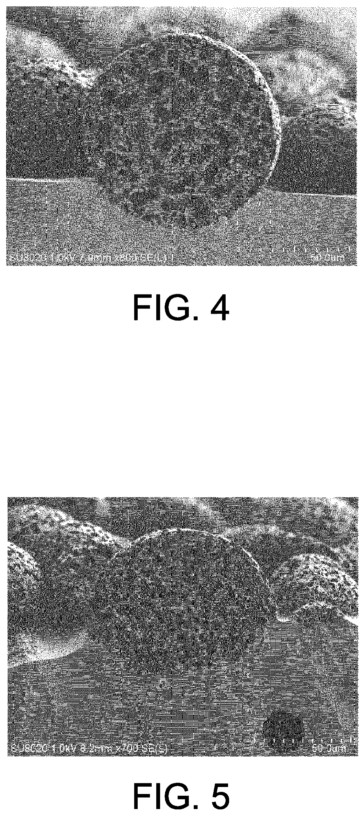

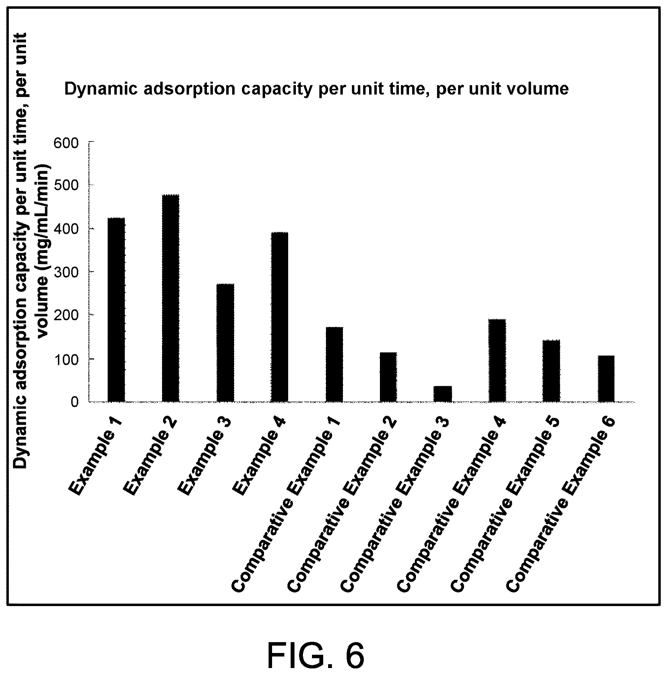

SEM observation was performed by using an ultra-high resolution field emission scanning electron microscope "SU8020" manufactured by Hitachi High-Technologies Corporation. As pretreatment, Au coating was applied to the frozen and dried particles of Manufacturing Examples 1-3 for imaging. In an electron microscope photograph, it can be observed that the obtained particles are almost perfectly spherical, and interconnected pores exist on the surface and cross-section (FIG. 1 to FIG. 5).
The evaluation results of each Manufacturing Example, Example, and Comparative Example are shown in Table 3 to Table 5 and FIG. 6.
Table 3 shows the median diameter, pore diameter, particle internal porosity, and retention time of silica fine particles with a particle diameter of 100 nm for particles of Manufacturing Examples 1 to 7.

[Table 3]

[Table 3]

|  | Median diameter (μm) | Pore diameter $r_{pore}$ (nm) | Particle internal porosity $\varepsilon_p$ (%) | Retention time of 100 nm silica |
|---|---|---|---|---|
| Manufacturing Example 1 | 64 | 1609 | 78 | 18.7 |
| Manufacturing Example 2 | 78 | 1222 | 81 | 15.8 |
| Manufacturing Example 3 | 84 | 715 | 85 | 17.6 |
| Manufacturing Example 4 | 77 | 598 | 80 | 13.8 |
| Manufacturing Example 5 | 53 | 490 | 79 | 13.0 |
| Manufacturing Example 6 | 86 | 100 | 90 | 14.0 |
| Manufacturing Example 7 | 83 | 1025 | 82 | 15.8 |

Table 4 shows the median diameter, pore diameter, particle internal porosity, type of functional group having the modified ligand, sulfur content, 10% dynamic adsorption capacity of γ-globulin at column residence time of 7.5 seconds, and the dynamic adsorption capacity per unit time and per unit volume for Examples 1 to 4 and Comparative Examples 1 to 6.

[Table 4]

[Table 4]

|  | Median diameter (μm) | Pore diameter $r_{pore}$ (nm) | Particle internal porosity $\varepsilon_p$(%) | Ligand Type | Sulfur content (%) | Adsorption capacity (mg/mL) | Adsorption capacity per unit time, per unit volume (mg/mL/min) |
|---|---|---|---|---|---|---|---|
| Example 1 | 64 | 995 | 70 | Sulfonic acid | 3.4 | 56 | 423 |
| Example 2 | 74 | 792 | 71 | Sulfonic acid | 3.4 | 63 | 478 |
| Example 3 | 90 | 425 | 73 | Sulfonic acid | 3.1 | 36 | 270 |
| Example 4 | 88 | 907 | 83 | Sulfate ester | 1.4 | 52 | 391 |
| Comparative Example 1 | 81 | 188 | 73 | Sulfonic acid | 3.6 | 23 | 172 |
| Comparative Example 2 | 55 | 111 | 63 | Sulfonic acid | 3.6 | 15 | 114 |
| Comparative Example 3 | 86 | 78 | 88 | Sulfonic acid | 28 | 5 | 38 |
| Comparative Example 4 | 53 | 187 | 81 | Sulfonic acid | 1.8 | 25 | 190 |
| Comparative Example 5 | 42 | 153 | 60 | Sulfonic acid | 0.9 | 19 | 142 |
| Comparative Example 6 | 86 | 20 | 86 | Sulfate ester | 3.1 | 14 | 108 |

FIG. 6 shows the 10% dynamic adsorption capacity per unit time and per unit volume of γ-globulin at the column residence time of 7.5 seconds when using the particles of Examples 1-4 and Comparative Examples 1-6.
As is evident from FIG. 6, Examples showed higher dynamic adsorption capacity per unit time and per unit volume than Comparative Examples.

[Industrial Applicability]

The invention describes a chromatography purification method at a high processing speed using porous particles having large interconnected pores forming a three-dimensional network-like skeleton and voids thereof, where the interconnected pores penetrate from the particle surface to the interior, and the porous particles are filled in a column. The invention can improve the amount of target substance bound per unit volume and per unit time (productivity) to enhance the separation and purification efficiency of biomedicines and the like, while utilizing conventional equipment such as filled columns and reducing the risk of clogging. Therefore, it is industrially very useful as it can achieve high throughput and high processing speed in the separation and purification processes of biomedicines, etc.

**Claims**

1. A porous particle,

   wherein the porous particle is substantially spherical and comprises an interconnected pore structure formed by a three-dimensional network skeleton and voids thereof, and the interconnected pores penetrate from a particle surface to an interior, and
   when the porous particle is filled in a column with an inner diameter of 0.78 cm and a length of 30 cm and a slurry containing 5 mg/mL of silica fine particles with a particle diameter of 100 nm is passed through using pure water as a mobile phase at a column temperature of 25°C and a flow rate of 0.4 mL/min, a retention time of the silica fine particles is 15.0 minutes or more.

2. The porous particle as claimed in claim 1, wherein a porous diameter is 650 nm or more.

3. A porous particle, modified by a ligand,

   wherein the porous particle is substantially spherical and comprises an interconnected pore structure formed by a three-dimensional network skeleton and voids thereof, and the interconnected pores penetrate from a particle surface to an interior, and
   a porous diameter is 400 nm or more.

4. The porous particle as claimed in any one of claims 1 to 3, wherein a main component is cellulose acetate or cellulose.

5. The porous particle as claimed in any one of claims 1 to 3, wherein a median diameter is 40 $\mu$m to 200 $\mu$m.

6. The porous particle as claimed in any one of claims 1 to 3, wherein a specific surface area according to a BET multipoint method is 1 m$^2$/g to 200 m$^2$/g.

7. The porous particle as claimed in claim 1 or 2, wherein the porous particle as claimed in claim 1 or 2 is modified by a ligand.

8. The porous particle as claimed in claim 7, wherein the ligand is one or more selected from a group consisting of a ligand containing an ion exchange group, an affinity ligand, and a ligand containing a hydrophobic group.

9. A liquid chromatography carrier, comprising the porous particle as claimed in any one of claims 1 to 3.

10. A liquid chromatography apparatus, comprising a separation column into which the liquid chromatography carrier as claimed in claim 9 is filled.

11. A separation and purification method of a biopolymer, comprising:
    a step of separating and purifying the biopolymer by using the liquid chromatography apparatus as claimed in claim 10.

12. The separation and purification method as claimed in claim 11, wherein a size of the biopolymer is 1 nm of 100 nm.

13. The separation and purification method as claimed in claim 11, wherein the biopolymer is an antibody.

FIG. 1

FIG. 2

FIG. 3

FIG. 4

FIG. 5

FIG. 6

## INTERNATIONAL SEARCH REPORT

| | International application No. |
|---|---|
| | **PCT/JP2024/007973** |

**A. CLASSIFICATION OF SUBJECT MATTER**

***B01J 20/282***(2006.01)i; ***B01J 20/285***(2006.01)i; ***G01N 30/88***(2006.01)i
FI: B01J20/282; B01J20/285 T; G01N30/88 D; G01N30/88 J

According to International Patent Classification (IPC) or to both national classification and IPC

**B. FIELDS SEARCHED**

Minimum documentation searched (classification system followed by classification symbols)

B01J20/282; B01J20/285; G01N30/88

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2009-510442 A (CENTER FOR APPLIED PROTEOMICS AND MOLECULAR MEDICINE) 12 March 2009 (2009-03-12) claims 1-3, 16, 20, 33, 36-37, 41, 50-51, paragraphs [0041], [0114] | 3, 7-13 |
| Y | | 1-13 |
| Y | JP 2014-178332 A (COVALENT MATERIALS CORP.) 25 September 2014 (2014-09-25) fig. 1-2, 8, paragraph [0077] | 1-13 |
| Y | JP 2021-506928 A (CYTIVA BIOPROCESS R & D AB) 22 February 2021 (2021-02-22) paragraphs [0016]-[0022] | 4-6 |
| A | JP 2020-026511 A (OSAKA GAS CHEMICALS CO., LTD.) 20 February 2020 (2020-02-20) entire text, all drawings | 1-13 |
| A | JP 2022-170261 A (TOKYO OHKA KOGYO CO., LTD.) 10 November 2022 (2022-11-10) entire text, all drawings | 1-13 |

☑ Further documents are listed in the continuation of Box C.    ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **18 April 2024** | **07 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
| --- |
| **PCT/JP2024/007973** |

### C. DOCUMENTS CONSIDERED TO BE RELEVANT

| Category* | Citation of document, with indication, where appropriate, of the relevant passages | Relevant to claim No. |
| --- | --- | --- |
| A | WO 2016/013568 A1 (DAICEL CORP.) 28 January 2016 (2016-01-28)<br>entire text, all drawings | 1-13 |
| A | WO 2017/026424 A1 (KYOTO UNIVERSITY) 16 February 2017 (2017-02-16)<br>entire text, all drawings | 1-13 |

Form PCT/ISA/210 (second sheet) (July 2022)

## INTERNATIONAL SEARCH REPORT
### Information on patent family members

International application No.

**PCT/JP2024/007973**

| Patent document cited in search report | | | Publication date (day/month/year) | Patent family member(s) | | | Publication date (day/month/year) |
|---|---|---|---|---|---|---|---|
| JP | 2009-510442 | A | 12 March 2009 | WO 2007/038523 A2 claims 1-3, 16, 20, 33, 36-37, 41, 50-51 US 2010/0240543 A1 EP 1929299 A1 | | | |
| JP | 2014-178332 | A | 25 September 2014 | US 2010/0021734 A1 fig. 1-2, 8 | | | |
| JP | 2021-506928 | A | 22 February 2021 | US 2021/0163529 A1 paragraphs [0018]-[0024] EP 3727680 A1 | | | |
| JP | 2020-026511 | A | 20 February 2020 | (Family: none) | | | |
| JP | 2022-170261 | A | 10 November 2022 | CN 117202984 A entire text, all drawings KR 10-2023-0174209 A | | | |
| WO | 2016/013568 | A1 | 28 January 2016 | US 2017/0210871 A1 entire text, all drawings EP 3173437 A1 | | | |
| WO | 2017/026424 | A1 | 16 February 2017 | US 2018/0223067 A1 entire text, all drawings EP 3327070 A1 | | | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016013568 A
- WO 2017026424 A
- JP 2020026511 A
- JP 2022515769 A
- WO 2015029790 A
- JP 2009242770 A

**Non-patent literature cited in the description**

- **W.M. DEEN ; F.G. SMITH III**. *J. Membr. Sci.*, 1982, vol. 12, 217-237
- **P DEPHILLIPS ; A M LENHOFF**. *J Chromatogr A.*, 2000, vol. 883, 39-54
- **J. K. ARMSTRONG et al.** The Hydrodynamic Radii of Macromolecules and Their Effect on Red Blood Cell Aggregation. *Biophysical Journal*, 2004, vol. 87, 4259-4270